# EUROPEAN PATENT SPECIFICATION

(11) **EP 3 297 993 B1**
(45) Date of publication and mention of the grant of the patent: **03.04.2019**
(21) Application number: 16724600.8
(22) Date of filing: 12.05.2016
(51) Int. Cl.: C07D 251/24

(54) **PROCESS FOR THE PREPARATION OF TRIAZINES**
VERFAHREN ZUR HERSTELLUNG VON TRIAZINEN
PROCÉDÉ POUR LA PRÉPARATION DES TRIAZINES

(30) Priority: 18.05.2015 WO PCT/EP2015/167969
(43) Date of publication of application: 28.03.2018
(73) Proprietor: DSM IP Assets B.V., 6411 TE Heerlen (NL)
(72) Inventor: GREINER, Nadine, 4303 Kaiseraugst (CH); SCHMID, Sandro, 4303 Kaiseraugst (CH); STEMMLER, René, Tobias, 4303 Kaiseraugst (CH)
(74) Representative: Berg, Katja
(86) International application number: PCT/EP2016/060617
(87) International publication number: WO 2016/184764

(56) References cited:
- US-A- 3 244 708
- US-A- 3 270 016
- US-A- 5 686 233
- US-A- 5 955 060

## Description

The invention relates to an improved process for the manufacture of bis-resorcinyl triazines of formula (I) wherein R¹ is a C₁-C₁₈alkyl group or C₂-C₁₈alkenyl group as well as the respective alkyl substituted bis-resorcinyl derivatives of formula (II) wherein R¹ is a C₁-C₁₈alkyl group or C₂-C₁₈alkenyl group and R² and R³ are independently of each other a C₁-C₁₈alkyl group or a C₂-C₁₈alkenyl group.

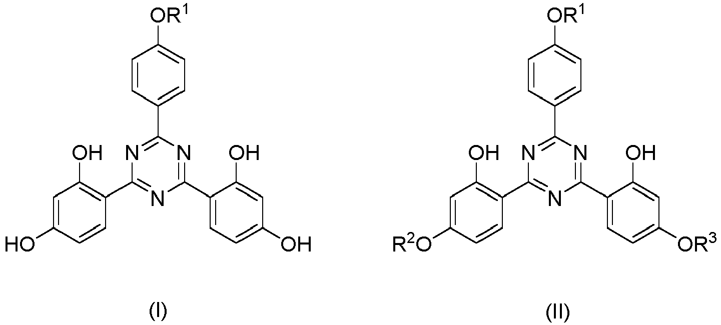

Bis-resorcinyl triazines of formula (I) are highly effective UV-absorbers which may, for example, be used as light stabilizers in plastics or as intermediates in the preparation of alkyl substituted bis-resorcinyl triazine derivatives of formula (II) such as for example Tinosorb® S [INCI Name: Bis-Ethylhexyloxyphenol Methoxyphenyl Triazine] which are particularly suitable as light screening agents in cosmetic products.

The preparation of bis-resorcinyl triazines of formula (I) and (II) is known and e.g. disclosed in US 5,955,060. The preparation encompasses the reaction of cyanuric chloride with a phenyl magnesium bromide compound in a Grignard reaction to a dichlorotriazine. The two resorcinyl groups are then introduced by a Friedel-Crafts acylation with resorcinol in the presence of a Lewis acid, in particular an aluminium halide. In a third step, the etherification of the free 4-hydroxyl groups is carried out by alkylation.

Because of the continuously increasing demand for bis-resorcinyl triazines based light screening agents the object of the present invention was to provide a process for the preparation of bis-resorcinyl triazines derivatives which is easy to carry out and affords economic advantages as a result of high yields. Furthermore, the disadvantage that the Friedel-Crafts acylation often yields unwanted by-products which are hardly removable and are subsequently carried over to the bis-resorcinyl triazines of formula (II) should be avoided.

Thus, the objective of the present invention was to provide a process for the preparation of bis-resorcinyl triazines of formula (I) and (II) which is easy to carry out and affords economic and regulatory advantages as a result of higher yields and higher purities.

Thus in a first aspect the present invention relates to a process (A) for the preparation of bis-resorcinyl triazines of formula (I), said process comprising the step of reacting a solution of dichlorotriazine of formula (III) in toluene, 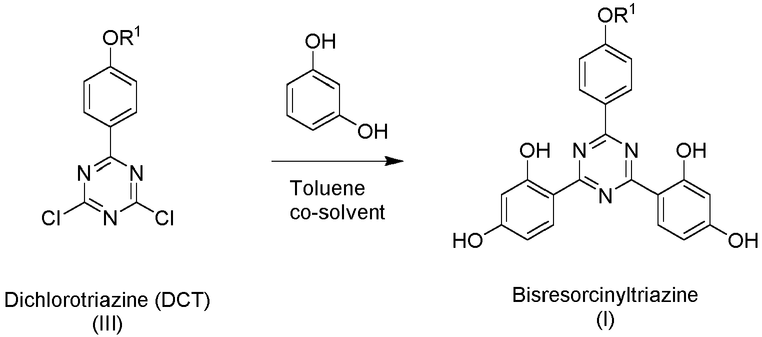 wherein R¹ is a C₁-C₁₈alkyl group or a C₂-C₁₈ alkenyl group,
with resorcinol in the presence of a Lewis acid and a co-solvent (Friedel-Crafts acylation), characterized in that the water content of the solution of the dichlorotriazine of formula (III) in toluene is less than 0.04 wt.-%, based on the total weight of the solution of the dichlorotriazine of formula (III) in toluene.

In the following, the dichlorotriazine of formula (III) is referred to as 'DCT' (DiChloroTriazine) and the solution thereof in toluene as 'DCT-toluene solution'.

In a preferred embodiment the water content of the DCT-toluene solution is less than 0.03 wt.-%, more preferably the water content of the DCT-toluene solution is less than 0.025 wt.-%, most preferably equal or less than 0.02 wt.-%, based on the total weight of the DCT-toluene solution.

Examples of C₁-C₁₈alkyl groups or C₂-C₁₈alkenyl groups are branched or unbranched alkyl, respectively alkenyl groups such as methyl, ethyl, n-propyl, 1-methylethyl, n-butyl, 1-methylpropyl-, 2-methylpropyl, 1,1-dimethylethyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, n-heptyl, 2-ethylhexyl, n-octyl, n-nonyl, n-decyl, n-undecyl, n-dodecyl, ethenyl, 2-propenyl and 3-butenyl groups.

In a preferred embodiment R¹ is a C₁-C₅alkyl group, more preferably a C₁-C₂alkyl group, most preferably a methyl group.

The term co-solvents as used herein refers to chemicals characterized by their ability to be miscible with toluene. Particular suitable co-solvents in all embodiments of the present invention encompass sulfolane, benzonitrile, chlorobenzene, nitrobenzene, acetonitrile and pivalonitrile as well as mixtures thereof. In all embodiments of the present invention it is preferred that only one co-solvent is used. Preferably the one co-solvent is selected from the group consisting of nitrobenzene, pivalonitrile and benzonitrile. Most preferably the co-solvent is nitrobenzene or benzonitrile as this leads to particular high yields.

Suitable Lewis acids encompass aluminium halides as well as magnesium halides. In all embodiments of the present invention the preferred Lewis acid is aluminium trichloride (AlCl₃).

In a particular advantageous embodiment, the invention encompasses a process (B), which is a process (A) wherein R¹ is a methyl group, the co-solvent is benzonitrile and the Lewis acid is aluminum trichloride.

In all embodiments of the present invention, the amount of resorcinol is at least 2 mol-equivalents with respect to the dichlorotriazine of formula (III). Preferably, a slight excess of resorcinol is used. Most preferably the amount of resorcinol is selected in the range of 2 to 2.5 mol-equivalents, with respect to the dichlorotriazine of formula (III).

In all embodiments of the present invention, the amount of the co-solvent is preferably selected in the range of 0.5 to 10, more preferably in the range of 1 to 6, most preferably in the range of 2 to 5 mol-equivalents, with respect to the dichlorotriazine of formula (III).

In all embodiments of the present invention, the amount of the Lewis acid is preferably selected in the range of 0.5 to 7, more preferably in the range of 0.75 to 5, most preferably in the range of 1 to 3 mol-equivalents, with respect to the dichlorotriazine of formula (III).

In all embodiments of the invention, the reaction temperature of the Friedel-Crafts acylation is preferably selected in the range of 25 C to 100°C such as more preferably in the range of 50°C to 70°C and most preferably in the range of 55° to 65°C (at atmospheric pressure). It is well understood that the reaction temperature would have to be adjusted accordingly if pressure/ vacuum is applied in the process according to the present invention, which however can easily be adjusted by a person skilled in the art and is encompassed herein as well.

Thus, in another particular advantageous embodiment, the invention encompasses a process (C), which is a process (B), wherein the amount of the co-solvent is selected in the range of 2 to 5 mol-equivalents with respect to the dichlorotriazine of formula (III), the amount of Lewis acid is selected in the range of 1 to 3 mol-equivalents with respect to the dichlorotriazine of formula (III) and the reaction temperature is selected in the range of 55° to 65°C (at atmospheric pressure).

It is furthermore preferred that the amount of dichlorotriazine of formula (III) in the DCT-toluene solution used in the processes according to the present invention is selected in the range of 5 to 25 wt.-%, preferably in the range of 10 to 20 wt.-%, most preferably in the range of 12 to 17 wt.-%, based on the total weight of the DCT-toluene solution.

Thus, in a very advantageous embodiment the invention encompasses a process (D) which is a process (C), wherein the DCT-toluene solution has a DCT content in the range of 5 to 25 wt.-%, preferably in the range of 10 to 20 wt, based on the total weight of the DCT-toluene solution.

The DCT-toluene solution having a water content as specified herein can be prepared by dissolving a dichlorotriazine of formula (III) in dry toluene (e.g. Toluene puriss. p.a., ACS reagent, ≥99.7% (GC) commercially available from Sigma-Aldrich) according to procedures well known to a person skilled in the art. Preferably, the dichlorotriazine of formula (III) used for preparing the solution has a purity (GC) of ≥ 90%, preferably of ≥ 94%, most preferably of ≥ 98%. If necessary the dichlorotriazine of formula (III) can be dried by vacuum-drying before dissolution according to standard methods in the art. A particular suitable dichlorotriazine of formula (III) which can be used for preparing the DCT-toluene solution according to the present invention is 2,4-Dichloro-6-(4-methoxyphenyl)-1,3,5-triazine [CAS 90723-86-7] having a purity (GC) of ≥ 94%, which is e.g. commercially available at Aldlab Chemical Building Blocks.

Alternatively and preferably, the solution of dichlorotriazine of formula (III) in toluene (DCT-toluene solution) is, however, prepared by a process (E) comprising the subsequent steps of
(i) Grignard reaction of cyanuric chloride with a 4-alkoxyphenylmagnesium halide of formula (IV) 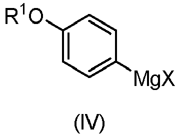 wherein R¹ is a C₁-C₁₈alkyl group or a C₂-C₁₈ alkenyl group and X is Cl, Br or I in tetrahydrofuran (THF) to yield a dichlorotriazine of formula (III), followed by
(ii) solvent exchange of THF with toluene resulting in the DCT-toluene solution, followed by
(iii) washing of the DCT-toluene solution with aqueous hydrochloric acid, followed by
(iv) phase separation and optionally a pre-drying of the DCT-toluene solution with a drying agent, followed by
(v) co-evaporation of the washed and optionally pre-dried DCT-toluene solution with toluene until a water content of less than 0.04 wt.-%, preferably less than 0.03 wt.-%, more preferably less than 0.025 wt.-%, most preferably equal or less than 0.02 wt.-% and a THF content of less than 3 wt.-%, preferably less than 2.5 wt.-%, most preferably less than 2 wt.-%, based on the total weight of the DCT-toluene solution is obtained.

In a particular advantageous embodiment, the DCT-toluene solution used in the process according to the invention has a water content of less than 0.04 wt.-% and a THF content of less than 3 wt.-%, more preferably a water content of less than 0.03 wt.-%, and a THF content of less than 2.5 wt.-% and most preferably a water content of less than 0.025 wt.-%, and a THF content of less than 2 wt.-%, such as a water content equal or less than 0.02 wt.-% and a THF content of less than 2 wt.-, based on the total weight of the DCT-toluene solution.

The drying agent in step (iv) can be selected from conventional drying agents commonly used in the organic laboratories such as the anhydrous forms of calcium chloride (CaCl₂), sodium sulfate (Na₂SO₄) calcium sulfate (CaSO₄ (as Drierite)) and magnesium sulfate (MgSO₄) as well as mixtures thereof. Preferably the drying agent is sodium sulfate (Na₂SO₄) or magnesium sulfate (MgSO₄).

In a very advantageous embodiment the invention encompasses a process (F), which is a process (D), wherein the DCT-toluene solution is prepared according to the process (E).

The water content ≥ 0.1 wt.-% as well as the THF content of the DCT-toluene solution of this invention is to be understood as determined by GC-TCD (i.e. a GC connected to a thermal conductivity detector). Water contents ≤ 0.1 wt.-% are determined by coulometric Karl-Fischer titration, as the latter method has a lower limit of quantification.

A particular advantageous 4-C₁-C₁₈alkoxyl- or C₂-C₁₈ alkenyloxyphenylmagnesium halogenide to be used in the Grignard reaction according to the present invention is 4-methoxyphenylmagnesium bromide, which can be prepared from 4-bromoanisol and magnesium turnings in THF according to standard methods in the art or is e.g. commercially available from Sigma-Aldrich (CAS 13139-86-1, 0.5 M in THF).

As aqueous hydrochloric acid preferably 1N HCl is used.

In a further embodiment, the process according to the present invention comprises a subsequent alkylation step of the bis-resorcinyl triazines of formula (I) with a C₁-C₁₈alkyl or C₂-C₁₈alkenyl halogenide to obtain the respective alkyl substituted bis-resorcinyl derivatives of formula (II). Preferably a C₃-C₁₀alkyl halogenide, more preferably a C₃-C₁₀alkyl halogenide and most preferably ethylhexyl halogenide is used. In a most preferred embodiment the bis-resorcinyl derivatives of formula (II) wherein R¹ is a methyl group and R² and R³ are a ethylhexyl groups is prepared according to the process of the present invention. The alkylation can be done according to standard methods in the art e.g. by etherification of a bis-resorcinyl triazines of formula (I) with an ethylhexyl halogenide such as 3-bromoethylhexane or 3-chloroethylhexane in the presence of a base as e.g. outlined in US 5,955,060 examples 1 and 2.

Each reaction of the process according to the invention can in principle be carried out in any reactor suitable for the respective reaction type. Without restricting generality, the following are mentioned by way of example: suspension reactor, stirred tank, stirred tank cascade, tubular reactor, shell-type reactor, shell and tube reactor, fixed-bed reactor, fluidized-bed reactor, reactive distillation column.

### Experimental part

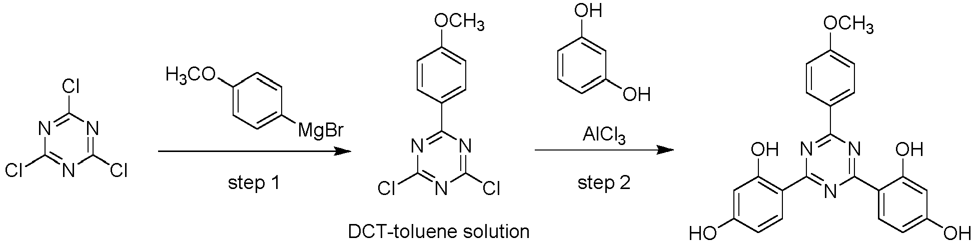

### Step 1: Synthesis of 2,4-dichloro-6-(4-methoxyphenyl)-1,3,5-triazine toluene solution

In a 500 mL 4-necked flask equipped with argon inlet, mechanical stirrer, reflux condenser, thermometer and addition funnel, 11.90 g magnesium turnings (0.488 mol, 1.20 eq) and a few grains of iodine were suspended in 130 mL dry THF. Under dry protective gas (nitrogen or argon) 5% of a solution of 92.2 g 4-bromoanisol (61.7 mL, 0.488 mol, 1.20 eq.) in 116 mL dry THF was added. An exothermic reaction indicated the start of the Grignard reaction, after which the remaining solution of 4-bromoanisol was added slowly. After complete addition and dissolution of the magnesium turnings, the jacketed reactor was heated to 70 °C and stirred for 2-4 h until all magnesium turnings dissolved and full conversion of 4-bromoanisol was observed (by GC analysis).

The resulting Grignard solution was then added dropwise at 0-5 °C to a suspension of 74.9 g cyanuric chloride (0.407 mol, 1.0 eq.) in 103 mL dry THF. After complete addition, the reactor was warmed to 25 °C and the reaction mixture was stirred for another 30 min. Then, vacuum was applied (-300 mbar) and the reactor was heated to 50 °C in order to distill off about 140 ml of THF. Subsequently, 700 mL of toluene was added continuously while distilling off a mixture of THF/toluene. Then 500 mL 1N HCl was added slowly. The phases were separated, furnishing 707 g of a solution of 2,4-dichloro-6-(4-methoxyphenyl)-1,3,5-triazine (DCT/M) in toluene (approx. 14.7 wt% DCT/M).

The water, respectively THF content of the resulting DCT/M-toluene solutions obtained as outlined above was adjusted by further co-evaporation with dry toluene (Fluka, puriss .P.A. ACS reagent) resulting in the DCT/M-toluene solutions as outlined in table 1, which were used in the subsequent reaction step 2.

**Table 1: Solutions of 2,4-dichloro-6-(4-methoxyphenyl)-1,3,5-triazine in toluene (DCT/M-toluene solutions)**

| ***Example*** | DCT/M [wt.-%] | THF [wt.-%] | H₂O [wt.-%] |
|---|---|---|---|
| ***Ref 1*** | 14.7 | 5.7 | 0.07 |
| ***Ref 2*** | 14.7 | 5.8 | 0.17 |
| ***Ref 3*** | 14.7 | 6.0 | 0.17 |
| ***Ref 4*** | 14.7 | 1.2 | 0.06 |
| ***1*** | 14.7 | 2.0 | 0.02 |
| ***2*** | 14.7 | None | 0.02 |

| | | | |
|---|---|---|---|
| The THF and H₂O content was determined by GC-TCD. A H₂O content below 0.1wt.-% was additionally analyzed by coulometric Karl-Fischer titration. | | | |

### Step 2: Synthesis of 4,4'-[6-(4-methoxyphenyl)-1,3,5-triazine-2,4-diyl]-bis-1,3-benzenediol

In a 1.5L glass reactor with argon inlet mechanical stirrer, reflux condenser, thermometer and an off-gas connection to a NaOH, 94 g of resorcinol (0.857 mol, 2.1 eq. with respect to DCT/M) and 3.2 mol eq. of benzonitrile (with respect to DCT/M) were added to 707g of the respective solution of DCT/M in toluene as outlined in table 1. The resulting solution was heated to 60 °C. Then 112 g AlCl₃ (0.842 mol, 2.07 eq. with respect to DCT/M) was added in portions. After complete addition the reaction was kept at 60 °C for approx. 4 h. Upon full conversion heating was stopped. Then 55 mL 1N HCI (55 mmol) was added dropwise, followed by 150 mL toluene, 165 mL 1N HCl (165 mmol) and 150 mL of water. The resulting suspension was filtered and subsequently washed with 350 mL toluene and 1000 mL of water. The filter cake was sucked dry and then dried under vacuum (-100 mbar) at 60 °C overnight, furnishing the indicated yield of 4,4'-[6-(4-methoxyphenyl)-1,3,5-triazine-2,4-diyl]-bis-1,3-benzenediol (MTB).

**Table 2: Comparison of yields and impurities using DCT/M-toluene solutions having different H₂O / THF contents**

| ***Example*** | DCT/M solution used | Impurity MW 311 [wt%] | Yield* MTB [wt%] |
|---|---|---|---|
| ***Ref 5*** | Ref 1 | 2.30 | 73 |
| ***Ref 6*** | Ref 2 | 2.41 | 71 |
| ***Ref 7*** | Ref 3 | 2.19 | 63 |
| ***Ref 8*** | Ref 4 | 0.71 | 76 |
| ***3*** | 1 | 0.13 | 81 |
| ***4*** | 2 | 0.29 | 84 |

| | | | |
|---|---|---|---|
| *based on cyanuric chloride | | | |

As can be retrieved from table 2, the yield of the 2,4-dichloro-6-(4-methoxyphenyl)-1,3,5-triazine (compound of formula (I) wherein R¹ is a methyl group) is significantly increased by reducing the water as well as the THF content in the DCT/M-solution. Furthermore, the impurity with a molecular weight of 311 g/mol (impurity MW 311) was significantly reduced.

Example 4 as outlined above was repeated by replacing toluene in example 1 by xylene and benzonitrile by sulfonlane (Ref 9), respectively by replacing benzonitrile by a different co-solvent (examples 5, 7 and 8) as outlined in table 3.

**Table 3: Comparison different solvents/ co-solvents**

| ***Example*** | DCT/M-solvent solution (14.7 wt.-%) | | | Co-solvent | Yield* MTB [wt%] |
|---|---|---|---|---|---|
| | Solvent | Water [ppm] | THF [ppm] | | |
| ***Ref 9*** | xylene | 0.028 | None | sulfolane | 74 |
| ***5*** | toluene | 0.022 | None | sulfolane | 78 |
| ***6*** | toluene | 0.020 | None | benzonitrile | 84 |
| ***7*** | toluene | 0.022 | None | nitrobenzene | 87 |
| ***8*** | toluene | 0.035 | None | pivalonitrile | 80 |

| | | | | | |
|---|---|---|---|---|---|
| *based on cyanuric chloride | | | | | |

As can be retrieved from table 3, the use of toluene led to an increase of the overall isolated yield. Furthermore, the use of benzonitrile, nitrobenzene respectively pivalonitrile as co-solvent is particularly advantageous.

## Claims

1. A process for the preparation of bis-resorcinyl triazines of formula (I) 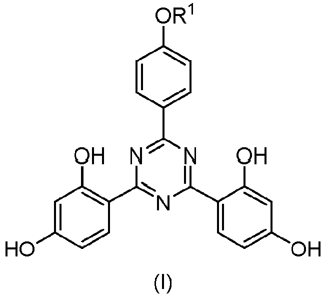 wherein R¹ is a C₁-C₁₈alkyl group or a C₂-C₁₈alkenyl group,
said process comprising the step of reacting a solution of dichlorotriazine of formula (III) 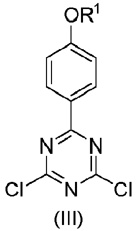 in DCT-toluene solution, DCT being dichlorotriazine with resorcinol in the presence of a Lewis acid and a co-solvent, **characterized in that** the water content of the DCT-toluene solution is less than 0.04 wt.-%, based on the total weight of the DCT-toluene solution.

2. The process according to claim 1, **characterized in that** the water content of the DCT-toluene solution is less than 0.03 wt.-%, based on the total weight of the DCT-toluene.

3. The process according to claim 1, **characterized in that** the water content of the DCT-toluene solution is less than 0.025 wt.-%, preferably equal or less than 0.02 wt.-%.

4. The process according to any one of claims 1 to 3, **characterized in that** R¹ is a C₁-C₂alkyl group.

5. The process according to claim 4, **characterized in that** R¹ is a methyl group.

6. The process according to any one of claims 1 to 5, **characterized in that** the Lewis acid is aluminium trichloride.

7. The process according to any one of claims 1 to 6, **characterized in that** the co-solvent is selected from the group consisting of benzonitrile, nitrobenzene and pivalonitrile.

8. The process according to any one of claims 1 to 7, **characterized in that** the amount of dichlorotriazine of formula (III) in the DCT-toluene solution is selected in the range of 5 to 25 wt.-%, based on the total weight of the DCT-toluene solution.

9. The process according to any one of claims 1 to 8, **characterized in that** the solution of dichlorotriazine of formula (III) in toluene (DCT-toluene solution) is prepared by
(i) Grignard reaction of cyanuric chloride with a 4-alkoxyphenylmagnesium halide of formula (IV) 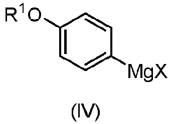 wherein R¹ is a C₁-C₁₈alkyl group or a C₂-C₁₈ alkenyl group, and X is Cl, Br or I
in tetrahydrofuran (THF) to yield a dichlorotriazine of formula (III), followed by
(ii) solvent exchange of THF with toluene resulting in a DCT-toluene solution, followed by
(iii) washing of the DCT-toluene solution with aqueous hydrochloric acid, followed by
(iv) phase separation and optionally a pre-drying of the DCT-toluene solution with a drying agent, followed by
(v) co-evaporation of the washed DCT-toluene solution with toluene until a water content of less than 0.04 wt.-%, preferably less than 0.03 wt.-%, more preferably less than 0.025 wt.-%, most preferably equal or less than 0.02 wt.-% and a THF content of less than 3 wt.-%, preferably less than 2.5 wt.-%, most preferably less than 2 wt.-%, based on the total weight of the DCT-toluene solution is obtained.

10. The process according to claim 9, **characterized in that** the solution of dichlorotriazine of formula (III) in toluene has a water content of less than 0.04 wt.-% and a THF content of less than 3 wt.-%,

11. The process according to claim 9, **characterized in that** the solution of dichlorotriazine of formula (III) in toluene has a water content of less than 0.03 wt.-% and a THF content of less than 2 wt.-%.

12. The process according to anyone of claims 9 to 11, **characterized in that** the 4-alkoxyphenylmagnesium halide is 4-methoxyphenylmagnesium bromide.

13. The process according to anyone of claims 9 to 12, **characterized in that** the drying agent is selected from the group consisting of the anhydrous forms of calcium chloride, sodium sulfate, calcium sulfate and magnesium sulfate as well as mixtures thereof.

14. The process according to any one of claims 1 to 13, **characterized in that** the bis-resorcinyl triazine of formula (I) is in a subsequent step etherified with an alkylhalogenide in the presence of a base.

15. The process according to claim 14, **characterized in that** the alkylhalogenide is 3-bromoethylhexane or 3-chloroethylhexane.

## Patentansprüche

1. Verfahren zur Herstellung von Bisresorcinyltriazinen der Formel (I) 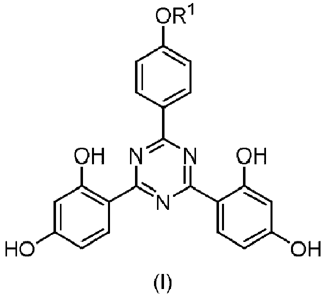 wobei R¹ für eine C₁-C₁₈-Alkylgruppe oder eine C₂-C₁₈-Alkenylgruppe steht,
wobei das Verfahren den Schritt des Umsetzens einer Lösung von Dichlortriazin der Formel (III) 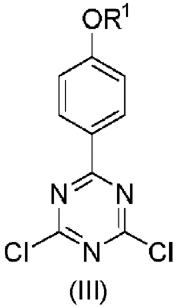 in DCT-Toluol-Lösung, wobei DCT für Dichlortriazin steht, mit Resorcinol in Gegenwart einer Lewis-Säure und eines Cosolvens umfasst, **dadurch gekennzeichnet, dass** der Wassergehalt der DCT-Toluol-Lösung weniger als 0,04 Gew.-%, bezogen auf das Gesamtgewicht der DCT-Toluol-Lösung, beträgt.

2. Verfahren nach Anspruch 1, **dadurch gekennzeichnet, dass** der Wassergehalt der DCT-Toluol-Lösung weniger als 0,03 Gew.-%, bezogen auf das Gesamtgewicht der DCT-Toluol-Lösung, beträgt.

3. Verfahren nach Anspruch 1, **dadurch gekennzeichnet, dass** der Wassergehalt der DCT-Toluol-Lösung weniger als 0,025 Gew.-%, vorzugsweise gleich oder weniger als 0,02 Gew.-%, beträgt.

4. Verfahren nach einem der Ansprüche 1 bis 3, **dadurch gekennzeichnet, dass** R¹ für eine C₁-C₂-Alkylgruppe steht.

5. Verfahren nach Anspruch 4, **dadurch gekennzeichnet, dass** R¹ für eine Methylgruppe steht.

6. Verfahren nach einem der Ansprüche 1 bis 5, **dadurch gekennzeichnet, dass** es sich bei der Lewis-Säure um Aluminiumtrichlorid handelt.

7. Verfahren nach einem der Ansprüche 1 bis 6, **dadurch gekennzeichnet, dass** das Cosolvens aus der Gruppe bestehend aus Benzonitril, Nitrobenzol und Pivalonitril ausgewählt wird.

8. Verfahren nach einem der Ansprüche 1 bis 7, **dadurch gekennzeichnet, dass** die Menge von Dichlortriazin der Formel (III) in der DCT-Toluol-Lösung im Bereich von 5 bis 25 Gew.-%, bezogen auf das Gesamtgewicht der DCT-Toluol-Lösung, ausgewählt wird.

9. Verfahren nach einem der Ansprüche 1 bis 8, **dadurch gekennzeichnet, dass** die Lösung von Dichlortriazin der Formel (III) in Toluol (DCT-Toluol-Lösung) durch
(i) Grignard-Reaktion von Cyanurchlorid mit einem 4-Alkoxyphenylmagnesiumhalogenid der Formel (IV) 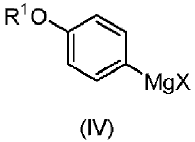 wobei R¹ für eine C₁-C₁₈-Alkylgruppe oder eine C₂-C₁₈-Alkenylgruppe steht und
X für Cl, Br oder I steht,
in Tetrahydrofuran (THF) zum Erhalt eines Dichlortriazins der Formel (III) gefolgt von
(ii) Lösungsmittelaustausch von THF mit Toluol, was eine DCT-Toluol-Lösung ergibt, gefolgt von
(iii) Waschen der DCT-Toluol-Lösung mit wässriger Salzsäure gefolgt von
(iv) Phasentrennung und gegebenenfalls Vortrocknen der DCT-Toluol-Lösung mit einem Trockenmittel gefolgt von
(v) Coverdampfung der gewaschenen DCT-Toluol-Lösung mit Toluol bis zum Erhalt eines Wassergehalts von weniger als 0,04 Gew.-%, vorzugsweise weniger als 0,03 Gew.-%, weiter bevorzugt weniger als 0,025 Gew.-%, ganz besonders bevorzugt gleich oder weniger als 0,02 Gew.-% und eines THF-Gehalts von weniger als 3 Gew.-%, vorzugsweise weniger als 2,5 Gew.-%, ganz besonders bevorzugt weniger als 2 Gew.-%, bezogen auf das Gesamtgewicht der DCT-Toluol-Lösung,
hergestellt wird.

10. Verfahren nach Anspruch 9, **dadurch gekennzeichnet, dass** die Lösung von Dichlortriazin der Formel (III) in Toluol einen Wassergehalt von weniger als 0,04 Gew.-% und einen THF-Gehalt von weniger als 3 Gew.-% aufweist.

11. Verfahren nach Anspruch 9, **dadurch gekennzeichnet, dass** die Lösung von Dichlortriazin der Formel (III) in Toluol einen Wassergehalt von weniger als 0,03 Gew.-% und einen THF-Gehalt von weniger als 2 Gew.-% aufweist.

12. Verfahren nach einem der Ansprüche 9 bis 11, **dadurch gekennzeichnet, dass** es sich bei dem 4-Alkoxyphenylmagnesiumhalogenid um 4-Methoxyphenylmagnesiumbromid handelt.

13. Verfahren nach einem der Ansprüche 9 bis 12, **dadurch gekennzeichnet, dass** das Trockenmittel aus der Gruppe bestehend aus den wasserfreien Formen von Calciumchlorid, Natriumsulfat, Calciumsulfat und Magnesiumsulfat sowie Mischungen davon ausgewählt wird.

14. Verfahren nach einem der Ansprüche 1 bis 13, **dadurch gekennzeichnet, dass** das Bisresorcinoltriazin der Formel (I) in einem nachfolgenden Schritt mit einem Alkylhalogenid in Gegenwart einer Base verethert wird.

15. Verfahren nach Anspruch 14, **dadurch gekennzeichnet, dass** es sich bei dem Alkylhalogenid um 3-Bromethylhexan oder 3-Chlorethylhexan handelt.

## Revendications

1. Procédé de préparation de bis-résorcinyltriazines de formule (I) 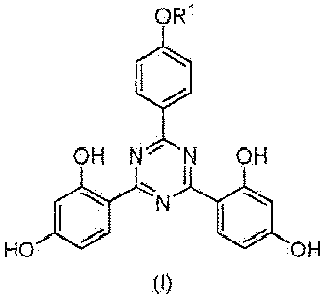 dans laquelle R¹ est un groupe alkyle en C₁-C₁₈ ou un groupe alcényle en C₂-C₁₈,
ledit procédé comprenant l'étape de réaction d'une solution de dichlorotriazine de formule (III) 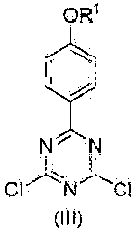 dans une solution de DCT-toluène, DCT étant la dichlorotriazine avec du résorcinol en présence d'un acide de Lewis et d'un cosolvant, **caractérisé en ce que** la teneur en eau de la solution de DCT-toluène est inférieure à 0,04 % en poids, sur la base du poids total de la solution de DCT-toluène.

2. Procédé selon la revendication 1, **caractérisé en ce que** la teneur en eau de la solution de DCT-toluène est inférieure à 0,03 % en poids, sur la base du poids total de DCT-toluène.

3. Procédé selon la revendication 1, **caractérisé en ce que** la teneur en eau de la solution de DCT-toluène est inférieure à 0,025 % en poids, de préférence égale ou inférieure à 0,02 % en poids.

4. Procédé selon l'une quelconque des revendications 1 à 3, **caractérisé en ce que** R¹ est un groupe alkyle en C₁-C₂.

5. Procédé selon la revendication 4, **caractérisé en ce que** R¹ est un groupe méthyle.

6. Procédé selon l'une quelconque des revendications 1 à 5, **caractérisé en ce que** l'acide de Lewis est le trichlorure d'aluminium.

7. Procédé selon l'une quelconque des revendications 1 à 6, **caractérisé en ce que** le cosolvant est choisi dans le groupe constitué du benzonitrile, du nitrobenzène et du pivalonitrile.

8. Procédé selon l'une quelconque des revendications 1 à 7, **caractérisé en ce que** la quantité de dichlorotriazine de formule (III) dans la solution de DCT-toluène est choisie dans la plage de 5 à 25 % en poids, sur la base du poids total de la solution de DCT-toluène.

9. Procédé selon l'une quelconque des revendications 1 à 8, **caractérisé en ce que** la solution de dichlorotriazine de formule (III) dans du toluène (solution de DCT-toluène) est préparée par
(i) réaction de Grignard de chlorure cyanurique avec un halogénure de 4-alcoxyphénylmagnésium de formule (IV) 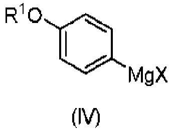 dans laquelle R¹ est un groupe alkyle en C₁-C₁₈ ou un groupe alcényle en C₂-C₁₈, et X est Cl, Br ou I
dans du tétrahydrofurane (THF) pour obtenir une dichlorotriazine de formule (III), suivie de
(ii) un échange de solvant de THF avec du toluène pour obtenir une solution de DCT-toluène, suivi de
(iii) un lavage de la solution de DCT-toluène avec de l'acide chlorhydrique aqueux, suivi de
(iv) une séparation de phase et facultativement une prédéshydratation de la solution de DCT-toluène avec un agent déshydratant, suivie de
(v) une coévaporation de la solution de DCT-toluène lavée avec du toluène jusqu'à ce qu'une teneur en eau inférieure à 0,04 % en poids, de préférence inférieure à 0,03 % en poids, plus préférablement inférieure à 0,025 % en poids, de manière préférée entre toutes égale ou inférieure à 0,02 % en poids et une teneur en THF inférieure à 3 % en poids, de préférence inférieure à 2,5 % en poids, de manière préférée entre toutes inférieure à 2 % en poids, sur la base du poids total de la solution de DCT-toluène soient obtenues.

10. Procédé selon la revendication 9, **caractérisé en ce que** la solution de dichlorotriazine de formule (III) dans du toluène a une teneur en eau inférieure à 0,04 % en poids et une teneur en THF inférieure à 3 % en poids.

11. Procédé selon la revendication 9, **caractérisé en ce que** la solution de dichlorotriazine de formule (III) dans du toluène a une teneur en eau inférieure à 0,03 % en poids et une teneur en THF inférieure à 2 % en poids.

12. Procédé selon l'une quelconque des revendications 9 à 11, **caractérisé en ce que** l'halogénure de 4-alcoxyphénylmagnésium est le bromure de 4-méthoxyphénylmagnésium.

13. Procédé selon l'une quelconque des revendications 9 à 12, **caractérisé en ce que** l'agent déshydratant est choisi dans le groupe constitué des formes anhydres de chlorure de calcium, sulfate de sodium, sulfate de calcium et sulfate de magnésium ainsi que des mélanges de ceux-ci.

14. Procédé selon l'une quelconque des revendications 1 à 13, **caractérisé en ce que** la bis-résorcinyltriazine de formule (I) est, dans une étape suivante, éthérifiée avec un halogénure d'alkyle en présence d'une base.

15. Procédé selon la revendication 14, **caractérisé en ce que** l'halogénure d'alkyle est le 3-bromoéthylhexane ou le 3-chloroéthylhexane.
